(19) Europäisches Patentamt
European Patent Office
Office européen des brevets

(11) Numéro de publication: 0 094 726
B1

(12) # FASCICULE DE BREVET EUROPÉEN

(45) Date de publication du fascicule du brevet: 15.01.86

(21) Numéro de dépôt: 83200685.2

(22) Date de dépôt: 16.05.83

(51) Int. Cl.⁴: C 07 D 301/02 // C07D303/04, C07D303/08, C07D303/22, C07D407/04, C07D409/04

(54) **Procédé de transformation d'un aldéhyde ou d'une cétone en époxyde correspondant, en particulier de furfural en furyloxirannes.**

(30) Priorité: 14.05.82 FR 8208631

(43) Date de publication de la demande: 23.11.83 Bulletin 83/47

(45) Mention de la délivrance du brevet: 15.01.86 Bulletin 86/3

(84) Etats contractants désignés: AT BE CH DE GB IT LI LU NL SE

(56) Documents cité:
FR-A-1 495 674
JOURNAL OF THE CHEMICAL SOCIETY, CHEMICAL COMMUNICATIONS, no. 15, 8 août 1973, pages 527-528, Londres, GB. Y. YANO et al.: "Oxiran formation in the base-catalysed reaction of long-chain alkyldimethylsulphonium salts and carbonyl compounds in benzene-water"
JOURNAL OF ORGANIC CHEMISTRY, vol. 34, no. 7, juillet 1969, pages 2133-2137, Columbus, Ohio, USA M.J. HATCH: "The synthesis of oxiranes from aqueous solutions of simple alkyl, allyl, and benzylsulfonium salts"
JOURNAL OF THE AMERICAN CHEMICAL SOCIETY, vol. 95, no. 16, 8 août 1973, pages 5311-5321, Columbus, Ohio, USA B.M. TROST et al.: "New synthetic reactions. Facile synthesis of oxaspiropentanes, versatile synthetic

(73) Titulaire: AGRIFURANE S.A., Z.I. de Boé Cidex 4412, F-47240 Bon Encontre (FR)

(72) Inventeur: Borredon, Elizabeth, F-46800 Montlauzun (FR)
Inventeur: Delmas, Michel, 23 Résidence des Amandiers Deyme, F-31450 Montgiscard (FR)
Inventeur: Gaset, Antoine, 75 allée de Brienne, F-31000 Toulouse (FR)

(74) Mandataire: Barre, Philippe, Cabinet Barre- Gatti-Laforgue 95 rue des Amidonniers, F-31069 Toulouse Cédex (FR)

(56) Documents cité: (suite)
intermediates"
ANGEWANDTE CHEMIE INTERNATIONAL EDITION, vol. 12, no. 10, octobre 1973, pages 845-846, Weinheim, DE. A. MERZ et al.: "Phase-transfer-catalyzed production of sulfur ylides in an aqueous system"

Il est rappelé que: Dans un délai de neuf mois à compter de la date de publication de la mention de la délivrance du brevet européen toute personne peut faire opposition au brevet européen délivré, auprés de l'Office européen des brevets. L'opposition doit être formée par écrit et motivée. Elle n'est réputée formée qu'après paiement de la taxe d'opposition (Art. 99(1) Convention sur le brevet européen).

EP 0 094 726 B1

LIBER, STOCKHOLM 1986

**Description**

L'invention concerne un procédé de transformation d'un aldéhyde ou d'une cétone ayant une ou plusieurs fonctions carbonyles, en époxyde correspondant (encore désigné par "oxiranne"); elle vise en particulier la transformation du furfural et de furylcétones.

A l'heure actuelle, le meilleur moyen connu pour obtenir industriellement un époxyde fait intervenir l'oxydation d'une double liaison et consiste à faire réagir un alcène avec une source d'oxygène en présence d'un catalyseur. Cette méthode, mise en oeuvre dans le seul secteur pétrochimique, souffre de graves limitations liées au contexte économique difficile propre aux produits pétroliers et surtout implique la production d'alcènes par voie pétrochimique, processus très lourd qui ne permet d'obtenir qu'une gamme très limitée de produits.

On connaît également d'autres méthodes pour fabriquer des époxydes, qui font appel à des composés carbonyles, mais ces méthodes ont toutes de graves inconvénients qui en rendent la mise en oeuvre industrielle problématique.

Une de ces méthodes consiste notamment à substituer un des hydrogènes en α de la fonction carbonyle par un halogène, à réduire par un hydrure la fonction carbonyle et à former l'époxyde par traitement en milieu basique de l'halohydrine obtenue. Le nombre d'étapes, le coût des réactifs et leur manipulation délicate, associés au faible rendement global obtenu en fin de réaction à partir de la fonction carbonyle, constituent des obstacles majeurs s'opposant au développement industriel d'un tel procédé.

Une autre méthode étudiée depuis 1968 met en oeuvre un processus de catalyse par transfert de phase utilisant un sel de sulfonium et un composé carbonylé. Cette technique implique la présence d'une phase aqueuse importante et corrosive, susceptible de générer de nombreuses réactions secondaires (réactions de Canizzaro, réaction d'auto-condensation suivie de déshydratation du substrat carbonylé etc...) ce qui limite considérablement l'intérêt de cette technique (Hatch, Journal of Organic Chemistry, 1969 - page 2133; TAKAGI et al, Chemical Communication, 1973 -page 527; MARKL et al, Angewandte Chemie, International Edition, 1973 - page 845). De plus, cette technique n'est pas applicable aux aldehydes ou cétones miscibles à l'eau comme l'acétaldehyde ou le furfural, ce qui constitue une limitation particulièrement gênante, car les époxydes obtenus à partir de ces dérives sont d'une importance industrielle considérable comme par exemple le méthyl-2 oxiranne ou oxyde de propylène.

Une autre méthode proposée a consisté à utiliser un polymère support qui sert à fixer le sel de sulfonium (technique développée depuis 1974), mais ce procédé implique l'usage de bases très fortes exigeant un milieu strictement anhydre, ce qui constitue un premier défaut grave de cette technique (FRECHET et al, Tetrahedron letters, 1979 page 203-206; Tetrahedron, 1981 - page 663). Le deuxième défaut majeur de cette technique qui, en soi, rend le développement industriel de celle-ci pratiquement impossible, reside dans l'utilisation de sels de thallium (composés très chers et d'une très haute toxicité), qui servent à fixer le sel de sulfonium sur la matrice polymérique.

Un autre procédé proposé par JOHNSON et al (Journal of American Chemical Society, 1961-page 417) et amélioré par COREY et al (Journal of the American Chemical Society 1965 - page 1353), puis par TROST et al (Journal of the American Chemical Society - Août 1973-page 5311-5321) consiste en une condensation entre un sel de sulfonium ou un sel d'oxosulfonium et un aldehyde ou une cétone en présence de bases fortes en milieu homogène liquide strictement anhydre. Les bases utilisées sont des bases fortes du type butyl-lithium, hydrures, alcoolates ou hydroxydes. L'obtention d'époxydes par cette réaction pose des problèmes tes difficiles à résoudre à l'échelle industrielle. En premier lieu, certaines bases fortes utilisées ont une réactivité violente et explosive au contact de l'eau, ce qui rend leur stockage dangereux. En outre et surtout, l'état strictement anhydre du milieu réactionnel exige, dans les installations industrielles, des traitements et contrôles spéciaux, qui sont très coûteux (étuvage, dessication, nécessité d'opérer sous courant d'azote... et ceci constitue un défaut majeur de ce type de procédé.

Le caractère strictement anhydre de ces réactions est une condition presentée comme fondamentale pour ce type de synthèse (ne faisant pas appel à un transfert de phase liquide/liquide); la littérature est abondante à cet égard et présente, toujours, comme particulièrement primordiale la nécessité de prévoir un milieu réactionnel rigoureusement anhydre:

- Dimethyloxosulfonium methylide [$(CH_3)2\ SOCH_2$] and Dimethylsulfonium methylide [$(CH_3)_2SCH_2$] - FORMATION AND APPLICATION TO ORGANIC SYNTHESIS - E.J. COREY and M. CHAYKOVSKY - JOURNAL OF AMERICAN CHEMICAL SOCIETY, 87, 1353 - 64, 1965.
- Transfer of alkyl substituted methyledes from sulfonium alkylides to carbonyl groups - E.J. COREY, W. OPPOLZER - JOURNAL OF AMERICAN CHEMICAL SOCIETY, 86, 1899 - 1900, 1964.
- Methylsulfinyl carbanion - Diméthylsulfoxonium méthylide-E.J. COREY, M. CHAYKOVSKY - JOURNAL OF AMERICAN CHEMICAL SOCIETY, 84, 866-8, 1962.
- Methylsulfinyl carbanium ($CH_3$-SO-$CH_2\ominus$) - Formation and Application to organic synthesis - E.J. COREY, M. CHAYKOVSKY - JOURNAL OF AMERICAN CHEMICAL SOCIETY, 87, 1345-53, 1965.
- Concerning the reaction of sulfonium methylides with conjugated carbonyl compounds - E.J. COREY, M. CHAYKOVSKY - TETRAHEDRON LETTERS, N° 4, 169-71, 1963.
- Dimethyl sulfonium methylide - A reagent for selective oxirane synthesis from aldehydes and Ketones - R. GREENWALD, M. CHAYKOVSKY - JOURNAL OF AMERICAN CHEMICAL SOCIETY, 84, 3782-3, 1962. - Chemistry of ylids: Diphenylsulfonium Alkylides - A stereo selective synthesis of epoxides - A.W. JOHNSON, V.J. KRUBY, J.L. WILLIAMS - JOURNAL OF AMERICAN CHEMICAL SOCIETY, 86, 918-22, 1963.

- The chemistry of ylids, XIX, β carbonylsulfonium ylides - A.W. JOHNSON, R.T. AMEL - JOURNAL OF ORGANIC CHEMISTRY, 34, 1240-7, 1969.

- The preparation and stereo specific rearrangement of spiro [bicyclo (2.2.1.)hept. 2. en -anti - 7,2' oxocyclopropane] the effect of a nonclassical intermediate - R.K. BLY and R.S. BLY - JOURNAL OF ORGANIC CHEMISTRY, 28, 3165-72, 1965.

- The mechanism of Nucleophilic alkylidene transfer by sulfonium and by sulfonium and oxosulfonium ylids - C.R. JOHNSON, C.W. SCHROECK, J.R. SHANKLIN - JOURNAL OF AMERICAN CHEMICAL SOCIETY, 95, 7424-31, 1973.

- Synthetic steroids - Part X - Preparation of (3S) and (3R) spiro - (5α- cholestane - 3, 2' - oxiran) and their corresponding 2α- methyl and 2,2 - dimethyl analogues - J. D. BALLANTINE, P.J. SYKES - JOURNAL OF CHEMICAL SOCIETY, 731-5, 1970.

- Umsetzung von sulfonium yliden mit polaren Doppelbindungen-V.FRANZEN, H.E. DRIESSEN - CHEMISCHE BERICHTE, 96, 1581-90, 1963. - Methyllenierung mit sulfonium yliden - V. FRANZEN, H. ERHARD-TETRAHEDRON LETTERS, N° 15, 661-2, 1962.

- Brevet canadien n° 1 090 797 - substituted 1 - thienyl and furyl - 2, 3, 4, 5 tetrahydro - 1H - 3 - benzazepine compounds-G. HOLDEN KENNETH, C. YIM NELSON - U.S.A.

- New Synthetic Reactions - Facile synthesis of oxaspiropentanes, versatile synthetic Intermediates - B.M. TROST, M.J. BOGDANOWIZ (+ réferences citées) - JOURNAL OF THE AMERICAN CHEMICAL SOCIETY, 95, 5311-21, 1973.

De tels travaux, qui s'étalent sur plus de 20 années, ont développé dans l'esprit du chimiste organicien un préjugé profondément enraciné concernant l'obligation rigoureuse d'un milieu réactionnel strictement anhydre. Cette règle a été élevée à la hauteur d'un principe de base de ce type de chimie.

C'est ainsi que, conformément à ce principe, B.M. TROST et son équipe, dont les travaux ont été évoqué plus haut, ont effectué une réaction de synthèse d'epoxyde dans un solvant formé par du D.M.S.O. (diméthylsulfoxyde), en veillant, de façon draconnienne, à ce que le milieu réactionnel soit totalement exempt d'eau.

Ce souci du caractère anhydre du milieu va si loin dans l'esprit des auteurs de l'article déjà mentionné (B.M. TROST, JOURNAL OF THE AMERICAN SOCIETY du 8 Août 1973 - page 5311) qu'ils prennent la précaution d'exécuter les opérations suivantes, préalablement à la réaction:

- séchage du D.M.S.O. sur hydrure de calcium (méthode de déshydratation extrêmement puissante et dangeureuse dont le mise en oeuvre est très délicate),
- séchage des appareillages à l'étuve ou sous azote,
- introduction des réactifs sous atmosphère anhydre azotée,
- déroulement de la réaction sous atmosphère anhydre azotée,
- séchage continu du solvant pendant la réaction au moyen de sulfate de magnésium anhydre.

Ces conditions expérimentales sont décrites en détail, en particulier pages 5318 et 5319, dans la publication sus-évoquée.

Il ressort donc de cet article et de toutes les publications antérieures, ayant trait à la synthèse d'époxyde sans transfert de phase liquide/liquide, que, à l'heure actuelle, il est tout à fait inconcevable, pour l'homme du métier, que de telles réactions de synthèse puissent se dérouler de façon satisfaisante en presence d'eau, et toutes les conditions contraignantes de déshydratation qui accompagnent ces procédés connus ne permettent pas de considérer ceux-ci comme des solutions industrielles possibles pour fabriquer des époxydes.

Dans ces conditions, à l'exception des procedés pétrochimiques, aucune solution parfaitement satisfaisante sur le plan industriel n'existe pour opérer cette fabrication, malgré l'abondance de la littérature sur ce sujet.

La présente invention se propose d'indiquer un nouveau procédé de transformation d'un aldéhyde ou d'une cétone du type dans lequel l'aldéhyde ou la cetone est mis en présence d'un sel de sulfonium et d'une base dans un solvant organique.

Un objectif essentiel de l'invention est de supprimer l'exigence stricte du caractère anhydre des réactions antérieures effectuées en milieu organique, en vue de fournir un procédé industriel bénéficiant d'une mise en oeuvre extrêmement facile et, donc, d'un faible coût, n'impliquant aucun danger ou risque particulier et susceptible de se dérouler dans des conditions douces de température et de pression.

Un autre objectif de l'invention est d'indiquer un procédé apte à opérer avec des solvants légers, peu onereux, susceptibles d'être régénérés et desquels les produits de synthèse sont faciles a séparer.

A cet effet, le procédé de transformation visé par l'invention est du type dans lequel l'aldéhyde ou la cétone est mis en présence d'un sel de sulfonium ou d'oxosulfonium et d'une base dans un solvant organique, la base utilisée étant une base minérale à l'état solide, de force basique en milieu aqueux, inférieure ou égale à celle de l'ion hydroxyde; selon la présente invention, l'on réalise la réaction dans les conditions suivantes: l'on ajuste l'hydratation du milieu réactionnel de façon à avoir un milieu non anhydre dont le taux d'hydratation est au plus égal à environ 10 moles d'eau par mole d'aldéhyde ou de cétone.

Les expérimentations ont montré, de façon inattendue, que l'utilisation d'une base minerale solide telle que définie ci-dessus (en particulier un hydroxyde alcalin ou alcalino-terreux) conjuguée à la mise en oeuvre d'un taux d'hydratation contrôlé conduit à un phénomène original de transfert solide/liquide, qui permet une transformation sélective de l'aldéhyde ou de la cétone en epoxyde avec de très hauts rendements. Le taux d'hydratation qui en particulier peut être ajusté entre 0,05 et 1 mole d'eau par mole d'aldéhyde ou de cétone, est très facile à contrôler et les conditions difficiles des réactions connues sont ainsi éliminées.

Ainsi, les inventeurs ont repoussé le préjugé, concernant le caractère strictement anhydre de ce type de réaction, et démontré que, de façon inattendue, la réaction de synthèse d'époxyde pouvait être menée en milieu anhydre avec un simple contrôle du taux d'hydratation (qui n'exige aucune précaution opératoire particulière); au surplus, et ceci est particulièrement remarquable, le rendement n'est pas affecté par les conditions opératoires de l'invention et se trouve du même ordre de grandeur que dans les procédés antérieurs.

Il est a noter que les réactions secondaires qui accompagnent généralement certains procédés connus et en abaissent le rendement (réaction de CANNIZZARO, aldolisation, formation d'alcool), sont inexistantes ou très limitées dans le procédé de l'invention et ne modifient pas de façon sensible son rendement.

Dans ces conditions, les inventeurs ont mis au point un procédé s'opposant aux idées reçues et, donc, à fortiori, non évident au vu de l'art antérieur, ce procédé constituant un progrès considérable en raison de ses avantages économiques par rapport aux procédés antérieurs:

- déroulement de la réaction en atmosphère libre,
- suppression de toutes les opérations préalables de déshydratation,
- supression au cours de la réaction, de la mise en oeuvre d'agents déshydratants,
- extraction simplifiée de l'époxyde synthétisé en raison de l'absence de corps étrangers,
- simplification considérable de l'appareillage de mise en oeuvre.

Par ailleurs, les expérimentations ont montré que la très grande majorité des sels de sulfonium ou d'oxosulfonium sont utilisables de sorte que le procédé permet de fabriquer des époxydes de structures très diverses; on peut notamment utiliser un sel de sulfonium ou d'oxosulfonium pourvu d'un groupement méthyl, de formule:

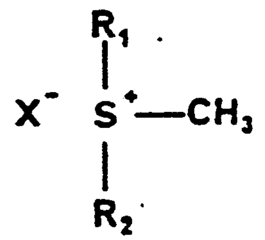

ou

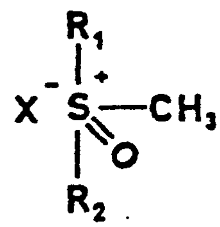

où X est un halogène et $R_1$ et $R_2$ sont des chaînes ou des cycles hydrocarbonés, saturés ou insaturés, fonctionnalisés ou non par un hétéroatome.

Ces sels permettent d'obtenir des époxydes substitués sur un des carbones du cycle oxygéné de l'époxyde.

On peut également utiliser un sel de silfonium ou d'oxosulfonium possédant un cycle soufré, de formule:

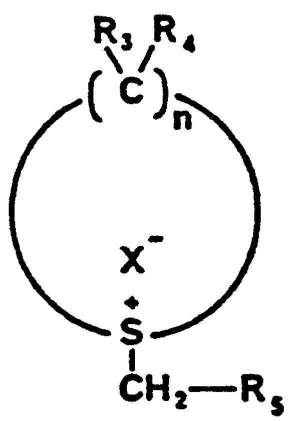

ou

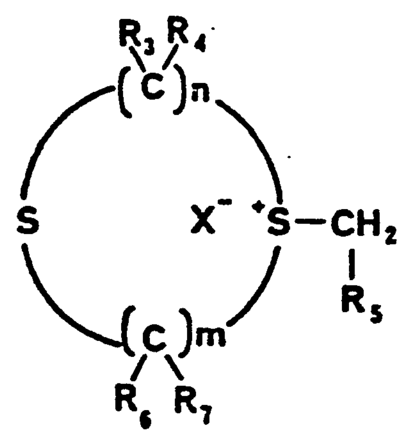

ou

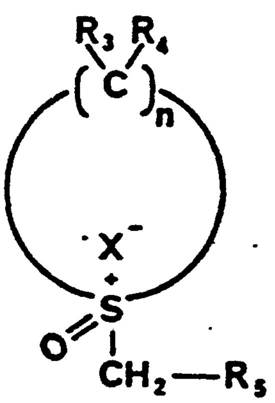

$1 < n < 10$

$1 \leq m < 8$

où X est un halogène, $R_3$, $R_4$, $R_5$, $R_6$ et $R_7$ sont des hydrogènes ou des chaînes ou cycles hydrocarbonés, saturés ou insaturés, fonctionnalisés ou non par un hétéroatome.

Ces sels permettent notamment d'obtenir des époxydes substitués sur les deux carbones du cycle oxygène de l'époxyde.

Par ailleurs, selon un mode de mise en oeuvre préféré, les réactifs sont mis en présence dans les conditions stoechiométriques suivantes:

. sel de sulfonium ou d'oxosulfonium dans un rapport approximativement stoechiométrique par rapport à l'aldéhyde ou à la cétone,

. base, sous forme solide, dans un rapport stoechiométrique approximativement compris entre 1 et 8 par rapport à l'aldéhyde ou à la cétone.

De plus, le procédé est avantageusement mis en oeuvre avec un solvant dissociant, peu ionisant, en particulier l'acétonitrile ou le nitrobenzène, qui sont très aisément recyclables.

La température a une influence très limitée sur la sélectivité de la réaction, mais une augmentation de température accroît sensiblement la vitesse de réaction. Dans la majorité des cas, le température optimale qui permet d'associer une bonne vitesse de réaction et un coût de mise en oeuvre modéré est voisine de 50° C. En pratique, le procédé sera mis en oeuvre a une température choisie dans une plage de température approximativement comprise entre la température ambiante et 60° C environ (dans un bon nombre de cas, le procédé peut être mis en oeuvre à température ambiante).

Le procédé de l'invention peut être appliqué comme l'illustrent les exemples qui suivent, au furfural à l'acétaldéhyde ou encore aux furylcétones, en vue de fabriquer les époxydes correspondants.

Il est à noter que le sulfure ou le sulfoxyde obtenu comme sous produit en fin de réaction peut être recyclé après un traitement avec un halogénure d'alkyle diversement substitué qui permet de régénérer le sel de sulfonium ou d'oxosulfonium.

De plus, les expérimentations ont montré que le procédé était applicable aux composés carbonyles portant une ou plusieurs fonctions aldéhyde ou cétone.

L'invention permet en particulier de synthétiser à partir du furfural ou de furylcétones, une molécule possédant le squelette suivant:

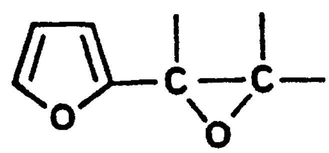

Le cycle furannique est connu pour apporter certaines propriétés caractéristiques aux composants qui l'intègrent (propriétés ignifuges, antibactériennes, fongicides etc...), propriétés dont bénéficient ces époxydes, ce qui leur ouvre de larges domaines d'application grâce à la combinaison de ces propriétés et de celles de la fonction époxyde en général.

L'invention exposée de façon générale ci-dessus est illustrée ci-après par des exemples non limitatifs de mise-en-oeuvre.

**EXEMPLE 1**

Synthèse du furyl-2 oxiranne à partir de furanne carboxaldéhyde-2 en présence d'iodure de triméthyl sulfonium et de potasse dans l'acétonitrile.

Dans un réacteur de 100 ml equipé d'un réfrigérant, d'un agitateur mécanique et d'une ampoule à addition, on place 0,02 mole. de sel de sulfonium, 0,12 mole de potasse en pastilles, 0,05 mole d'$H_2O$ dans 20 cc d'acétonitrile.

On chauffe la solution à 60° C pendant 2 heures pour former l'ylure de sulfonium.

On ajoute alors dans le réacteur 0,02 mole de furanne carboxaldéhyde-2 dilué dans 20 cc d'acétonitrile par l'intermédiaire de l'ampoule à addition.

Après une heure de réaction, la phase solide correspondant au mélange: iodure de potassium et hydroxyde de potassium est séparée par filtration et lavée à l'éther. Après evaporation du solvant de la solution organique, on récupère le furyl-2 oxiranne avec un rendement égal à 90 % environ.

Après purification sur une colonne de silice, il est analysé par chromatographie en phase gazeuse sur colonne OV17 et caractérisé par ses spectres 1R,RMN (H et $^{13}C$) dont les résultats sont confirmés par microanalyse.

Son point d'ébullition est de 56° C sous 14 mm de Hg (1841 pascals).

Le furyl-2 oxiranne conduit à la synthèse d'une résine thermoplastique adhésive après réaction avec de l'anhydride phtalique.

Le composé de départ: le furanne-carboxaldéhyde-2 (ou furfural) est fabriqué industriellement à partir de sous-produits agricoles riches en pentosanes (rafles de maïs, balles de riz et d'avoine, bagasses de canne à sucre, coques d'arachides et déchets de bois). Ces déchets traités en milieu acide concentré conduisent à des aldopentoses obtenus par hydrolyse de leurs pentosanes qui se deshydratent pour aboutir au furfural.

**EXEMPLE 2**

Synthèse du furyl-2 oxiranne à partir du furanne carboxaldéhyde-2 en présence d'iodure de méthylthiolanium et de potasse dans l'acétonitrile.

Dans un réacteur identique de 100 ml, on introduit 0,02 mole de sel de sulfonium, 0,16 mole de potasse en pastilles, 0,05 mole d'$H_2O$ dans 20 cc d'acétonitrile.

Le milieu réactionnel est mis en agitation à 60° C pendant 2 heures. On rajoute 0,02 mole de furanne carboxaldéhyde-2 dilué dans 20 cc d'acétonitrile.

Après une heure de réaction, on sépare la phase solide de la solution organique.

Après évaporation du solvant, le furyl-2 oxiranne est purifie sur une colonne de silice. On l'obtient avec un rendement de 80 %.

Il est caractérisé par ses spectres IR, RMN (du H, et du $^{13}C$) dont les résultats sont confirmés par micro-analyse.

Remarque: la base KOH peut être utilisée sous forme de pastilles ou en poudre.

**EXEMPLE 3**

Synthèse de furyl-2 méthyl-3 oxiranne à partir du furanne carboxaldéhyde-2 en présence d'iodure d'éthylthiolanium et de potasse dans l'acétronitrile.

Dans un réacteur de 100 ml on introduit: - 0,02 mole de sel de sulfonium

- 0,16 mole de potasse en pastilles
- 0,05 mole d'$H_2O$
- 20 cc d'acétronitrile

On mélange pendant 2 heures à 60°C. On ajoute ensuite 0,02 mole de furanne carboxaldéhyde-2 dilue dans 20 cc d'acétonitrile.

Une heure plus tard, on récupère la solution organique séparée de la phase solide.

Après évaporation du solvant et purification sur une colonne de silice, le furyl-2methyl-3oxiranne est recueilli avec un rendement de 75%.

Il est caractérisé par ses spectres IR, RMN (H et $^{13}C$) dont les résultats sont confirmés par microanalyse.

**EXEMPLE 4**

Synthèse du furyl-2 propyl-3 oxiranne à partir du furanne carboxaldéhyde-2 en présence d'iodure de butythiolanium et de potasse dans l'acétonitrile.

Dans un réacteur de 100 ml, on mélange 0,02 mole de sel de sulfonium, 0,12 mole de potasse en pastilles, 0,005 mole d'eau dans 20 cc d'acétonitrile.

Après 2 heures de formation de l'ylure de thiolanium à 60° C, on ajoute 0,02 mole de furanne carboxaldéhyde-2 dilué dans 20 cc d'acétonitrile. La réaction dure 1 heure.

Après filtration de la phase solide et évaporation du solvant, on recueille le furyl-2 propyl-3 oxiranne, on le purifie par un passage sur colonne de silice. Il est obtenu avec un rendement de 70% et caractérisé par ses spectres IR, RMN (H et $^{13}C$) dont les résultats sont confirmés par microanalyse.

**EXEMPLE 5**

Synthèse du phenyl-2 oxiranne à partir du benzaldéhyde en présence d'iodure de trimethylsulfonium et de $K_2CO_3$ dans le méthanol.

Dans un réacteur de 100 ml, on introduit 0,02 mole de sel de sulfonium, 0,04 mole de carbonate de potassium hydraté (contenant un molécule d'eau par molécule de carbonate) dans 20 cc de méthanol.

Après 2 heures de réaction à température ambiante, on ajoute 0,02 mole de benzaldéhyde dilué dans 20 cc de méthanol.

6 heures plus tard, on sépare par filtration la phase solide de la solution organique.

Après filtration du solvant, le phényl-2 oxiranne est purifié sur une colonne de silice. On le récupère avec un rendement de 55% et on le caractérisé par ses spectres IR, RMN (H et $^{13}C$) dont les résultats sont confirmés par microanalyse.

Son point d'ébullition est de 80°C sous 5 mm de Hg.

**EXEMPLE 6**

Synthèse du (nitro-2 phényl)-2 oxiranne à partir du nitro-2 benzaldéhyde et d'iodure de trimethylsulfonium avec KOH hydraté (même taux que précédemment) dans le diéthylèneglycoldiméthyléther ou "Diglyme".

Dans un réacteur de 100 ml on introduit:
- 0,02 mole de sel de sulfonium
- 0,04 mole de potasse en pastilles
- 20 cc de diglyme

On mélange pendant 2 heures à température ambiante. On ajoute ensuite 0,02 mole de nitro-2 benzaldéhyde dilué dans 20 cc de diglyme.

Six heures plus tard, on récupère la solution organique séparée de la phase solide.

Après évaporation du solvant et purification sur une colonne de silice, le [nitro-2 phényl] -2 oxiranne est recueilli avec un rendement de 85%.

Il est caractérisé par ses spectres IR, RMN (H et $^{13}C$) dont les résultats sont confirmés par microanalyse.

Ce produit permet la synthèse de dérivés hétérocycliques aromatiques homologues de l'indole par exemple.

**EXEMPLE 7**

Synthèse du [nitro-3 phényl] -2 oxiranne à partir du nitro 3 - benzaldéhyde et d'iodure de triméthylsulfonium avec de la potasse dans le dioxanne 1-4.

Dans un réacteur de 100 ml, on mélange 0,02 mole de sel de sulfonium, 0,04 mole de potasse en pastilles 0,025 mole d'eau dans 20 cc de dioxanne 1-4 pendant 2 heures a 40° C;

On ajoute dans le milieu réactionnel 0,02 mole de nitro-3 benzaldéhyde dilué dans 20 cc de de dioxanne 1-4.

Après 7 heures de réaction, on sépare la phase solide de la solution organique. On évapore le solvant de cette solution. Le [nitro-3 phényl]-2 oxiranne après purification sur colonne de silice est obtenu avec un rendement de 70.%. Il est caractérisé par ses spectres IR et RMN (H, $^{13}C$) dont les résultats sont confirmés par microanalyse.

**EXEMPLE 8**

Synthèse du [méthoxy-4 phényl]-2 oxiranne à partir du méthoxy-4 benzaldéhyde et d'iodure de triméthylsulfonium avec de la potasse dans l'acétonitrile.

Dans un réacteur de 100 ml, on introduit 0,02 mole de sel de sulfonium, 0,04 mole de potasse en pastilles, 0,025 mole d'eau dans 20 cc d'acétonitrile.

Après formation de l'ylure pendant 2 heures à 60° C, on ajoute 0,02 mole de methoxy-4 benzaldéhyde diluée dans 20 cc d'acétonitrile.

Six heures plus tard, on sépare par filtration la phase solide de la solution organique.

Après évaporation du solvant, le [méthoxy-4 phényl]-2 oxiranne est purifié sur une colonne de silice. On le récupère avec un rendement de 80% et on le caractérise par ses spectres IR, RMN (H et $^{13}C$) dont les résultats sont confirmés par microanalyse.

Le méthoxy-4 benzaldéhyde ou anisaldéhyde est un produit extrait de l'acacia mais peut aussi s'obtenir après méthylation d'aldéhyde phénolique issue de la lignine.

**EXEMPLE 9**

Synthèse du dioxiranne-yl 1-4 benzène à partir du téréphtaldéhyde, d'iodure de triméthylsulfonium et de potasse dans l'acétonitrile.

Dans un réacteur de 100 ml, on introduit:
- 0,02 mole de sel de sulfonium
- 0,12 mole de potasse en pastilles
- 0,015 mole d'eau
- 20 cc d'acétonitrile

On mélange pendant 2 heures à température ambiante. On ajoute ensuite 0,02 mole de téréphtaldéhyde diluée dans 20 cc d'acétonitrile.

Trois heures plus tard, on récupère la solution organique séparée de la phase solide.

Après évaporation du solvant et purification sur une colonne de silice, le dioxiranne-yl 1-4 benzène est recueilli avec un rendement de 80%.

Il est caractérisé par ses spectres IR, RMN (H et $^{13}C$) dont les résultats sont confirmés par microanalyse.

Il est à noter que cette molécule permet la reticulation de la cellulose et des polymères hydroxylés.

**EXEMPLE 10**

Synthèse du thiényl-2 oxiranne à partir du thiophène carboxaldéhyde-2, de bromure de triméthyl sulfonium et de potasse dans l'acétonitrile.

Dans un réacteur de 100 ml, on mélange 0,02 mole de sel dé sulfonium, 0,12 mole de potasse en poudre et 0,0 5 mole d'eau dans l'acétonitrile pendant 2 heures à température ambiante.

On ajoute dans le milieu réactionnel 0,02 mole de thiophène carboxaldéhyde-2 dilué dans 20 cc d'acétonitrile.

Apres 3 heures de réaction, on sépare la phase solide de la solution organique. On évapore le solvant et on recueille le thiényl-2 oxiranne après purification sur colonne de silice avec un rendement de 80%. Il est caractérisé par ses spectres IR et RMN (H et $^{13}C$) dont les résultats sont confirmés par microanalyse.

**EXEMPLE 11**

Synthèse du furyl-2 méthyl-2 oxiranne à partir de la furyl-2 méthyl cétone, d'iodure de triméthylsulfonium et de potasse dans le dioxanne 1-4.

Dans un reacteur de 100 ml, on introduit 0,02 mole de sel de sulfonium, 0,12 mole de potasse en pastilles, 0,05 mole d'eau, dans le dioxanne 1-4.

Après formation de l'ylure pendant 2 heures a 70°C, on ajoute 0,02 mole de furyl-2 méthyl cétone dilué dans 20 cc de dioxanne 1-4.

Une heure plus tard, on sépare par filtration la phase solide de la solution organique.

Après évaporation du solvant, le furyl-2 méhtyl-2 oxiranne est purifie sur une colonne de silice. On le récupère avec un rendement de 90% et on le caractérise par ses spectres IR et RMN (H, $^{13}C$) dont les résultats sont confirmés par la microanalyse.

**EXEMPLE 12**

Synthèse du phényl-2 méthyl-2 oxiranne à partir de l'acétophénone, d'iodure de triméthyl sulfonium et de potasse dans l'acétonitrile.

Dans un reacteur de 100 ml, on introduit:
- 0,02 mole de sel de sulfonium
- 0,12 mole de potasse en pastilles
- 0,05 mole d'eau
- 20 cc d'acétonitrile.

On mélange pendant 2 heures à température ambiante. On ajoute ensuite 0,02 mole d'acétophenone diluée dans 20 cc d'acétonitrile.

Quatre heures plus tard, on récupère la solution organique séparée de la phase solide.

Après évaporation du solvant et purification sur une colonne de silice, le phényl-2 méthyl-2 oxiranne est recueilli avec un rendement de 50%.

Il est caractérisé par ses spectres IR, RMN (H et $^{13}C$) dont les résultats sont confirmés par microanalyse.

**EXEMPLE 13**

Synthèse du propyl-2 méthyl-2 oxiranne à partir de la pentanone, d'iodure de triméthyl sulfonium et de potasse dans l'acétonitrile. Dans un réacteur de 100 ml, on mélange 0,02 mole de sel de sulfonium, 0,12 mole de potasse en pastilles, 0,05 mole d'eau dans 20 cc d'acétonitrile à 60° C pendant 2 heures.

On ajoute dans le milieu réactionnel: 0,02 mole de pentanone diluée dans 20 cc d'acétonitrile.

Apres 3 heures de réaction, on sépare la phase solide de la solution organique. On évapore le solvant et on recueille le propyl-2 méthyl-2 oxiranne après purification sur colonne de silice avec un rendement de 70%. Il est caractérisé par ses spectres IR et RMN (H et $^{13}C$) dont les résultats sont confirmés par microanalyse.

**EXEMPLE 14**

Synthèse du pentyl-2 oxiranne à partir de l'hexanal (ou capronaldéhyde), d'iodure de triméthyl sulfonium et de potasse dans le dioxanne 1-4.

Dans un reacteur de 100 ml, on introduit 0,02 mole de sel de sulfonium, 0,12 mole de potasse en pastilles, 0,05 mole d'eau, dans le dioxanne 1-4.

Après formation de l'ylure pendant 2 heures a 40° C, on ajoute 0,02 mole d'hexanal dilué dans 20 cc de dioxanne 1-4.

3 heures plus tard, on sépare par filtration la phase solide de la solution organique.

Après évaporation du solvant, le pentyl-2 oxiranne est purifié sur une colonne de silice. On le récupère avec un rendement de 60% et on le caractérisé par ses spectres IR et RMN (H et $^{13}C$) dont les résultats sont confirmés par la microanalyse.

**EXEMPLE 15**

Synthèse du décanyl-2 oxiranne a partir d'un décanal d'iodure de triméthylsulfonium et de potasse dans le dioxanne 1-4.

Dans un réacteur de 100 ml, on introduit:
- 0,02 mole de sel de sulfonium
- 0,012 mole de potasse en pastilles
- 0,025 mole d'eau
-20 cc de dioxanne 1-4

On mélange pendant 2 heures à 70° C.

On ajoute ensuite 0,02 mole d'undécanal diluée dans 20 cc de dioxanne 1-4.

Quatre heures plus tard, on récupère la solution organique séparée de la phase solide par filtration.

Après évaporation du solvant et purifition sur une colonne de silice le décanyl-2 oxiranne est recueilli avec un rendement de 70%.

Il est caractérisé par ses spectres IR, RMN (H et $^{13}C$) dont les résultats sont confirmés par microanalyse.

**EXEMPLE 16**

Synthèse du propyl-2 oxiranne à partir du butanal, d'iodure de triméthylsulfonium, de potasse dans le dioxanne 1-4.

Dans un réacteur de 100 ml, on mélange à température ambiante pendant 2 heures: 0,02 mole de sel de sulfonium, 0,01 mole d'eau, 0,12 mole de potasse en pastilles dans 20 $cm^3$ de dioxanne 1-4.

On ajoute dans le milieu réactionnel 0,02 mole de butanal diluée dans 20 cc de dioxanne 1-4.

Après 3 heures de réaction, on sépare la phase solide par filtration du milieu réactionnel.

On distille la solution recueillie et on recueille le propyl-2 oxiranne avec un rendement de 60%.

Son point d'ébullition est de 59° C sous 760 mm de Hg.

On caractérise le produit par ses spectres IR et RMN (H et $^{13}C$) dont les résultats sont confirmés par microanalyse.

**EXEMPLE 17**

Synthèse du phényl-2 oxiranne à partir du benzaldéhyde de chlorure de triméthyloxosulfonium et de potasse dans le diéthylène glycol diméthyl éther.

Dans un réacteur de 100 ml, on introduit 0,02 mole de sel d'oxosulfonium, 0,04 mole de potasse en pastilles, 0,05 mole d'eau dans 20 cc de diéthylène glycol dimethyl éther.

Après formation de l'ylure pendant 2 heures à 60° C, on ajoute 0,02 mole de benzaldéhyde diluée dans 20 cc de diéthylène glycol dimethyl éther.

3 heures plus tard, on sépare par filtration la phase solide du milieu réactionnel.

Après évaporation du solvant, le phényl-2- oxiranne est purifié sur une colonne de silice. On le récupère avec un rendement de 60% et on le caractérisé par ses spectres IR, RMN (H et $^{13}C$) dont les résultats sont confirmés par la microanalyse.

**Revendications**

1/ - Procédé de transformation d'un aldéhyde ou d'une cétone en époxyde correspondant, dans lequel l'aldéhyde ou la cétone est mis en présence d'un sel de sulfonium ou d'oxosulfonium et d'une base dans un solvant organique, la base utilisée étant une base minérale à l'état solide, de force basique en milieu aqueux, inférieure ou égale à celle de l'ion hydroxyde, ledit procédé étant caractérisé en ce que l'on réalise la réaction dans les conditions suivantes: l'on ajuste l'hydratation du milieu réactionnel de façon à avoir un milieu non anhydre dont le taux d'hydratation est au plus égal à environ 10 moles d'eau par mole d'aldéhyde ou de cétone.

2/ - Procédé de transformation selon la revendication 1, caractérisé en ce que l'on utilise comme base un hydroxyde alcalin ou alcalino-terreux.

3/ - Procédé de transformation selon l'une des revendications 1 ou 2, caractérisé en ce que:

. le sel de sulfonium ou d'oxosulfonium est mis en présence de l'aldéhyde ou de la cétone dans un rapport approximativement stoechiométrique par rapport à ces composés,

. et la base est mise en présence de l'aldéhyde ou de la cétone sous forme solide dans un rapport stoechiométrique approximativement compris entre 1 et 8 par rapport à ces composés.

4/ - Procéde de transformation selon l'une des revendications 1, 2 ou 3, caractérisé en ce que l'on ajuste le taux d'hydratation du milieu réactionnel à une valeur approximativement comprise entre 0,05 et 1 mole d'eau par mole d'aldéhyde ou de cétone.

5/ - Procédé de transformation selon l'une des revendications 1, 2, 3 ou 4, caractérisé en ce que l'on utilise un solvant dissociant, peu ionisant.

6/ - Procédé de transformation selon l'une des revendications 1, 2, 3, 4 ou 5, caractérisé en ce qu'il est mis en oeuvre à une température approximativement comprise entre la température ambiante et 60° C.

7/ - Procédé de transformation selon l'une des revendications 1, 2, 3, 4, 5 ou 6, caractérisé en ce que l'on utilise un sel de sulfonium ou d'oxosulfonium pourvu d'un groupement méthyl, de formule:

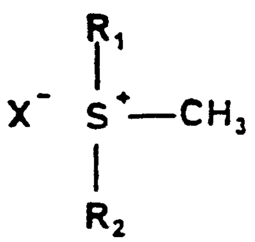

ou

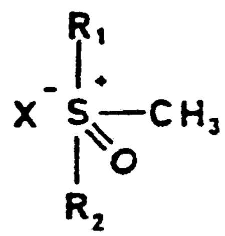

où X est un halogène et $R_1$ et $R_2$ sont des chaînes ou des cycles hydrocarbonés, saturés ou insaturés, fonctionnalisés ou non par un hétéroatome.

8/ - Procédé de transformation selon l'une des revendications 1, 2, 3, 4, 5 ou 6, caractérisé en ce que l'on utilise un sel de sulfonium ou d'oxosulfonium possédant un cycle soufré de formule:

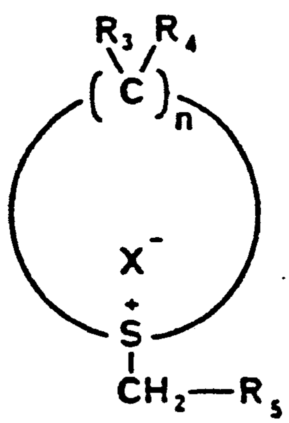

ou

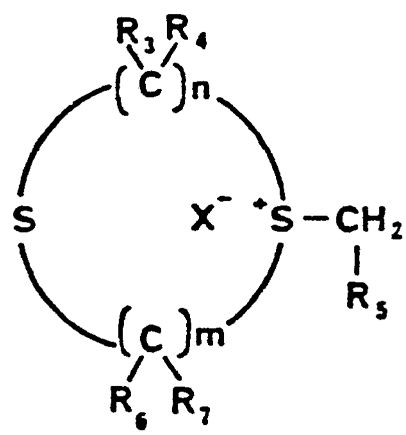

ou

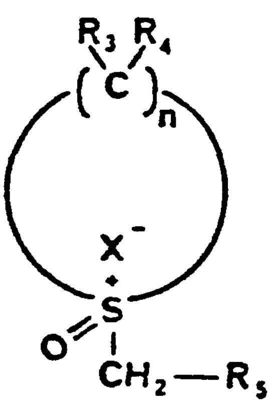

$1 < n < 10$

$1 \leq m < 8$

où X est un halogène, $R_3$, $R_4$, $R_5$, $R_6$, et $R_7$ sont des hydrogènes ou des chaînes ou cycles hydrocarbones, saturés ou insaturés, fonctionnalisés ou non par un hétéroatome.

9/ - Procédé selon l'une des revendications 1 à 8 appliqué au furfural en vue de la fabrication d'un époxyde du furfural.

10/ - Procedé selon l'une des revendications 1 à 8, appliqué à l'acétaldéhyde en vue de la fabrication d'un époxyde de l'acétaldéhyde.

11/ - Procédé selon l'une des revendications 1 à 8, appliqué à une furylcétone en vue de la fabrication d'un furyloxiranne.

**Claims**

1. Process for the transformation of an aldehyde or a ketone into the corresponding epoxide, in which the aldehyde or ketone is brought into contact with a sulphonium salt or oxosulphonium salt and a base in an organic solvent, the base used being an inorganic base in the solid state, having a basic strength in aqueous medium lower then, or equal to, that of the hydroxyl ion, said process being characterised in that the reaction is carried out in the following conditions: the water content of the reaction medium is adjusted so as to obtain a non-anhydrous medium, the water content level of which is, at most, equal to about 10 moles of water per mole of aldehyde or ketone.

2. Transformation process according to Claim 1, characterised in that the base used is an alkali metal hydroxide or alkaline erath metal hydroxide.

3. Transformation process according to either Claim 1 or 2, characterised in that

- the sulphonium salt or oxosulphonium salt is brought into contact with the aldehyde or ketone in an approximately stoichiometric ratio, with respect to these compounds, and
- the base is brought into contact with the aldehyde or ketone is solid form in a stoichiometric ratio ranging approximately from 1 to 8, with respect to these compounds.

4. Transformation process according to one of the Claims 1, 2 or 3, characterised in that the water content level of the reaction medium is adjusted to a value ranging approximately from 0.05 to 1 mole of water per mole of aldehyde or ketone.

5. Transformation process according to one of the Claims 1, 2, 3 or 4, characterised in that a dissociative, low ionising solvent is used.

6. Transformation process according to one of the Claims 1, 2, 3, 4 or 5, characterised in that it is carried out at a temperature ranging approximately from ambient temperature to 60°C.

7. Transformation process according to one of the Claims 1, 2, 3, 4, 5 or 6, characterised in that a sulphonium salt or oxosulphonium salt is used, containing a methyl group and the formula:

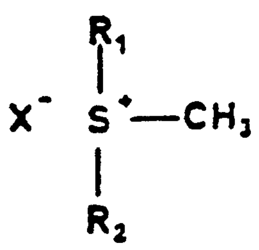

or

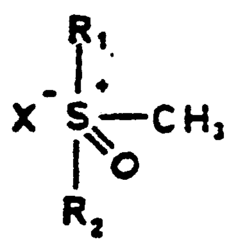

where X is a halogen and $R_1$, $R_2$, are hydrocarbon chains or rings, saturated or unsaturated, unsubstituted or substituted by a hetero-atom.

8. Transformation process according to one of the Claims 1, 2, 3, 4, 5 or 6, characterised in that a sulphonium salt or oxosulphonium salt is used, having a sulphur ring of the formula

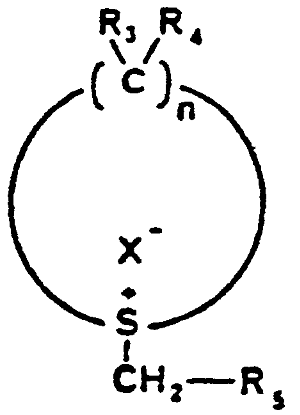

or

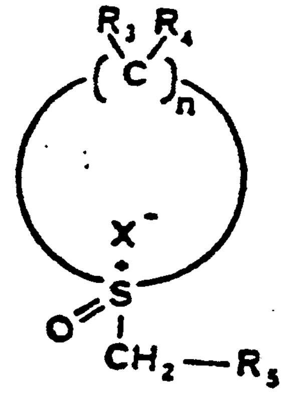

or

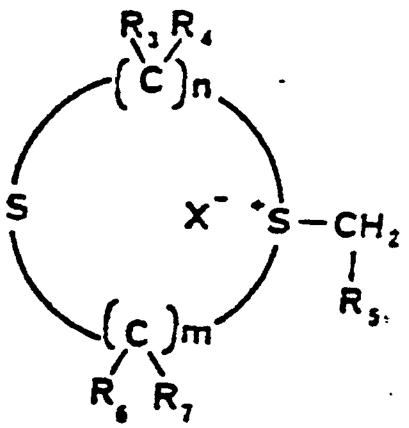

$1 < n < 10$

$1 \leqslant m < 8$

wher X is a halogen and $R_3$, $R_4$, $R_5$, $R_6$ and $R_7$, are hydrogen atoms or hydrocarbon chains or rings, saturated or unsaturated, unsubsitiuted or substituted by a hetero-atom.

9. Process according to one of the Claims 1 to 8, applied to furfural, with a view to producing an epoxide of furfural.

10. Process according to one of the Claims 1 to 8, applied to acetaldehyde, with a view to producing an epoxide of acetaldehyde.

11. Process according to one of the Claims 1 to 8, applied to a furyl ketone, with a view to producing a furyl oxirane.

**Patentansprüche**

1. Verfahren zur Umwandlung eines Aldehyds oder eines Ketons in das entsprechende Epoxid, bei welchem das Aldehyd oder Keton mit einem Sulfonium- oder Oxosulfoniumsalz und einer Base in einem organischen Lösungsmittel zusammengebracht wird, wobei es sich bei der verwendeten Base um eine anorganische Base in festem Zustand handelt, welche eine basische Stärke in wässrigem Medium aufweist, die geringer als die des Hydroxylions oder ihr gleich ist, wobei besagtes Verfahren dadurch gekennzeichnet ist, dass man die Umsetzung unter folgenden Bedingungen durchführt: man stellt den Wassergehalt des Reaktionsmediums so ein, dass man ein nicht wasserfreies Medium erhält, dessen Wassergehaltsspiegel höchstens etwa 10 Mol Wasser pro Mol Aldehyd oder Keton beträgt.

2. Umwandlungsverfahren nach Anspruch 1, dadurch gekennzeichnet, dass man als Base ein Alkali- oder Erdalkalihydroxyd verwendet.

3. Umwandlungsverfahren nach einem der Ansprüche 1 oder 2, dadurch gekennzeichnet, dass

- das Sulfonium- oder Oxosulfoniumsalz mit dem Aldehyd oder Keton in etwa stöchiometrischem Verhältnis hinsichtlich dieser Verbindungen zusammengebracht wird,
- die Base mit dem Aldehyd oder Keton in fester Form in stöchiometrischem Verhältnis etwa zwischen 1 und 8 hinsichtlich dieser Verbindungen zusammengebracht wird.

4. Umwandlungsverfahren nach einem der Ansprüche 1, 2 oder 3, dadurch gekennzeichnet, dass man den Wassergehaltsspiegel des Reaktionsmediums auf einen etwa zwischen 0.05 und 1 Mol Wasser pro Mol Aldehyd oder Keton liegenden Wert einstellt.

5. Umwandlungsverfahren nach einem der Ansprüche 1, 2, 3 oder 4, dadurch gekennzeichnet, dass man ein dissoziierendes, schwach ionisierendes Lösungsmittel verwendet.

6. Umwandlungsverfahren nach einem der Ansprüche 1, 2, 3, 4 oder 5, dadurch gekennzeichnet, dass es bei einer etwa zwischen der Umgebungstemperatur und 60°C liegenden Temperatur durchgeführt wird.

7. Umwandlungsverfahren nach einem der Ansprüche 1, 2, 3, 4, 5 oder 6, dadurch gekennzeichnet, dass man ein eine Methylgruppe enthaltendes Sulfonium- oder Oxosulfoniumsalz mit der Formel

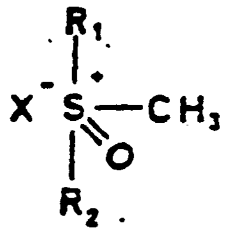

oder

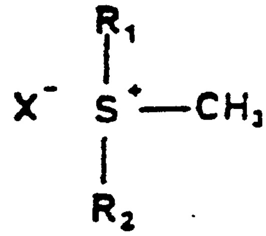

verwendet, wobei X ein Halogen ist und $R_1$ und $R_2$ gesättigte oder ungesättigte, unsubstituierte oder durch ein Heteroatom substituierte Kohlenwasserstoffketten oder -ringe sind.

8. Umwandlungsverfahren nach einem der Ansprüche 1, 2, 3, 4, 5 oder 6, dadurch gekennzeichnet, dass man ein Sulfonium- oder Oxosulfoniumsalz verwendet, welches einen Schwefelring besitzt mit der Formel

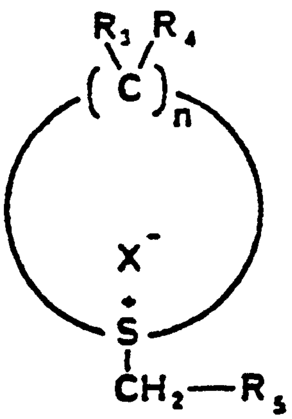

oder

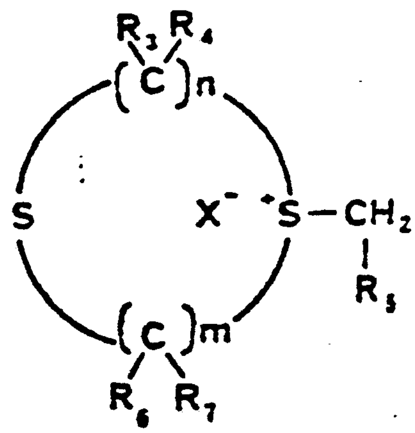

oder

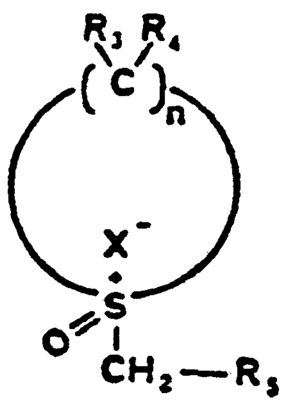

$1 < n < 10$

$1 \leqslant m < 8$ ,

wobei X ein Halogen ist und $R_3$, $R_4$, $R_5$, $R_6$ und $R_7$ Wasserstoffatome oder gesättigte oder ungesättigte, unsubstituierte oder durch ein Heteroatom substituierte Kohlenwasserstoffketten oder -ringe sind.

9. Verfahren nach einem der Ansprüche 1 bis 8 mit Anwendung auf Furfural zwecks Herstellung eines Epoxids des Furfurals.

10. Verfahren nach einem der Ansprüche 1 bis 8 mit Anwendung auf Acetaldehyd zwecks Herstellung eines Epoxids des Acetaldehyds.

11. Verfahren nach einem der Ansprüche 1 bis 8 mit Anwendung auf ein Furylketon zwecks Herstellung eines Furyloxirans.